# DEMANDE DE BREVET EUROPEEN

(11) **EP 1 216 658 A1**
(43) Date de publication de la demande: **26.06.2002**
(21) Numéro de dépôt: 01403340.1
(22) Date de dépôt: 21.12.2001
(51) Int. Cl.: A61B 5/103

(54) **Procédé pour déterminer un degré d'une caractéristique de la typologie corporelle**

(30) Priorité: 21.12.2000 FR 0016771
(71) Demandeur: L'OREAL, 75008 Paris (FR)
(72) Inventeur: Bazin, Roland, 91570 Bievres (FR)
(74) Mandataire: Tonnellier, Jean-Claude

(57) **Abrégé**

La présente invention concerne un procédé permettant de déterminer le degré effectif et/ou souhaité d'une caractéristique de la typologie corporelle d'un individu, caractérisé par le fait qu'il comporte les étapes suivantes :
a) générer une séquence d'images (21, 22,) exprimant ladite caractéristique avec un degré variable, notamment variant progressivement, et
b) permettre d'identifier à l'intérieur de la séquence, au moins une image ou suite d'images correspondant au degré effectif et/ou au degré souhaité de ladite caractéristique.

## Description

La présente invention est relative à l'évaluation de caractéristiques de la typologie corporelle, notamment en vue de l'établissement d'un diagnostic.

Certains sites accessibles sur Internet préconisent aux internautes des produits cosmétiques ou de soins à partir des réponses formulées par les internautes à des questionnaires.

Ainsi, certains sites recommandent des produits en fonction de l'âge des internautes et de la réponse à une question concernant le type de peau, à savoir par exemple sèche, normale, mixte ou grasse.

D'autres sites demandent encore la couleur des cheveux, aidant l'internaute dans son choix par l'affichage d'un nuancier.

D'autres sites encore demandent à l'internaute d'indiquer s'il a très peu, peu ou beaucoup de rides.

Hormis la caractéristique de couleur des cheveux, qui peut être évaluée dans une certaine mesure assez précisément à l'aide d'un nuancier affiché, les questionnaires disponibles sur les différents sites comportent des questions le plus souvent vagues, si bien qu'il est difficile pour l'internaute de choisir entre plusieurs réponses.

Il en résulte une grande imprécision sur l'évaluation des caractéristiques de la typologie corporelle des internautes.

Par "caractéristiques de la typologie corporelle", on entend au sens de la présente demande des caractéristiques mécaniques, morphologiques ou physiologiques, ainsi que des caractéristiques colorimétriques telles que la carnation ou la couleur des cheveux.

On connaît par la demande britannique GB 2 288 511 un procédé permettant d'établir un diagnostic à distance, consistant à envoyer une image d'une zone à diagnostiquer à un centre distant où un expert peut établir un diagnostic.

Un tel procédé mobilise un expert, ne permet pas une auto-évaluation, et la qualité du diagnostic dépend notamment de la qualité de l'image adressée à l'expert, donc de la prise de vue, ce qui le rend relativement difficile à mettre en oeuvre et peu fiable.

A la connaissance de la société déposante, pour évaluer précisément les caractéristiques de la typologie corporelle, notamment le relâchement du cou, la fermeté de la peau, la cellulite, la ptose ou les rides, on fait venir les personnes dans un centre d'examen où un professionnel tel qu'une esthéticienne les examine et procède à l'évaluation souhaitée.

Cette méthode génère des frais de transport, d'accueil et de rémunération des personnes s'étant déplacées.

De plus, les personnes examinées ne sont pas toujours les plus représentatives de la population générale, car domiciliées essentiellement dans les environs du centre d'examen, ce qui constitue une barrière géographique gênante pour une évaluation objective.

Il est encore connu, pour l'évaluation de l'état de la chevelure par exemple, de couper une mèche et de l'envoyer à un centre de diagnostic où un expert peut l'examiner.

Cette méthode n'est pas très pratique et le diagnostic est relativement long à établir compte tenu des délais postaux.

Il existe un besoin pour évaluer de manière précise une caractéristique de la typologie corporelle tout en permettant de réduire le coût de l'évaluation.

Il existe également un besoin pour établir rapidement un diagnostic, sans l'aide d'un professionnel, à distance si cela est souhaité.

Il existe encore un besoin pour permettre de constituer facilement et rapidement une banque de données de la typologie corporelle.

Il existe enfin un besoin pour mettre en évidence les premiers signes d'amélioration suite à un traitement cosmétique, afin d'encourager la personne à poursuivre le traitement, et le cas échéant pour déterminer l'efficacité d'un produit cosmétique ou de soins.

L'invention vise à répondre à tout ou partie de ces besoins.

Elle y parvient grâce à un nouveau procédé permettant de déterminer le degré effectif et/ou souhaité d'une caractéristique de la typologie corporelle d'un individu, ce procédé étant caractérisé par le fait qu'il comporte les étapes suivantes :
a) générer une séquence d'images exprimant la caractéristique avec un degré variant d'une image à l'autre, notamment variant progressivement et,
b) permettre d'identifier à l'intérieur de la séquence, au moins une image ou suite d'images correspondant au degré effectif et/ou au degré souhaité de ladite caractéristique.

Par "images" au sens de la présente invention, on entend toute image affichée ou projetée, 2D ou 3D, ou toute image non affichée, correspondant à des données numériques stockées dans une mémoire ou sur un support d'enregistrement de données numériques, tel qu'un disque dur ou un cédérom.

Par « séquence » au sens de la présente invention, on entend une série d'images qui, lorsqu'elles sont visualisées, ne le sont pas toutes de manière simultanée.

En particulier, lorsque les images sont visualisées, elles le sont de préférence image par image.

Les images de la séquence correspondent aux différents degrés de la caractéristique de la typologie corporelle de préférence selon une progression continue ou sensiblement continue, c'est-à-dire qu'un observateur observant deux images successives de la séquence ne distingue pas de différence sensible entre elles.

Chaque image peut correspondre à la représentation d'une zone du corps, maquillée ou non, traitée ou non, ou à la représentation d'un état d'un dispositif de test, par exemple un ruban adhésif connu sous la dénomination commerciale D'SQUAM ®.

La caractéristique de la typologie corporelle objet de la séquence d'images peut être choisie parmi les suivantes :
- caractéristiques relatives au vieillissement telles que nombre et profondeur des rides, relâchement de la peau, notamment du cou, nombre et profondeur des plis, notamment du cou, plissement de la peau, profondeur des cernes, taux de cellulite, ptose,
- caractéristiques physiologiques ou morphologiques telles que nombre et taille des cornéoparticules de la peau, taux de sécrétion de sébum, taux de sécrétion de la sueur, sécheresse de la peau ou des lèvres, longueur, courbure ou densité des cils ou des cheveux, irrigation sanguine de la peau, défaut de pigmentation, densité de points noirs, de boutons d'acné, de couperose, grains de la peau, contour des lèvres,
- couleur de la peau ou des cheveux, cette liste n'étant pas limitative.

Grâce à l'invention, on peut notamment déterminer précisément le degré d'une caractéristique de la typologie corporelle chez une personne que l'on cherche à évaluer, en particulier lorsque l'évolution de cette caractéristique d'une image à l'autre au sein de la séquence d'images est progressive, de préférence continue ou sensiblement continue.

La séquence d'images peut être générée aisément sur un ordinateur personnel, au moyen d'un serveur ou sur un dispositif de conseil présent sur un lieu de vente par exemple, et permet notamment à une personne de s'auto-évaluer sans la présence d'un professionnel.

L'évaluation peut en outre être indépendante de conditions de prise de vue, puisqu'elle peut se faire, selon un aspect de l'invention, sans l'acquisition d'une image de la personne que l'on cherche à évaluer.

Le procédé selon l'invention est avantageusement mis en oeuvre, comme indiqué plus haut, pour permettre à des personnes de s'auto-évaluer, sans l'aide d'une personne spécialement formée à cet effet.

Il peut encore être mis en oeuvre pour permettre à une personne de déterminer un degré souhaité d'une caractéristique de la typologie corporelle, ce degré étant destiné à servir par exemple d'objectif à atteindre à l'issu d'un traitement.

Il peut encore être mis en oeuvre pour mettre en évidence une amélioration au cours d'un traitement ou mesurer l'efficacité d'un produit.

Par ailleurs, le procédé selon l'invention permet une évaluation de la typologie corporelle pratiquement sans limites concernant le nombre de personnes pouvant être consultées.

L'invention permet notamment de constituer aisément et rapidement une banque de données importante, à partir d'un groupe comportant par exemple plus de quatre-vingt personnes, voire encore nettement plus, par exemple plus de mille personnes.

L'évaluation peut s'effectuer à distance, au domicile même des personnes à évaluer.

Ainsi, toute barrière géographique est supprimée.

L'évaluation pouvant avoir lieu au domicile de chaque personne, il devient possible d'évaluer des personnes qu'il aurait été impossible de faire se déplacer à un centre d'examen, notamment des personnes à pouvoir d'achat élevé qu'une rémunération versée en contrepartie d'une évaluation effectuée dans un centre d'examen ne peut nullement motiver.

Selon un aspect de l'invention, les images de la séquence d'images sont affichées sur un écran et présentées à une personne afin de permettre à celle-ci de sélectionner une image, et l'on génère une information représentative de l'image sélectionnée, notamment à distance au moyen d'Internet, par exemple.

Une telle information représentative de l'image sélectionnée peut comporter par exemple un nombre, ce dernier pouvant se présenter par exemple sous la forme d'une note proportionnelle au nombre d'images compris entre l'une des images correspondant à un degré extrême de la caractéristique de la typologie corporelle en cause et l'image sélectionnée.

Le procédé peut ainsi comporter, selon un aspect de l'invention, les étapes consistant à afficher en un premier emplacement géographique des images d'une séquence d'images générée conformément à l'invention, puis à transférer par exemple sous un ou plusieurs protocoles de communication, notamment sous protocole IP, des informations représentatives d'une sélection opérée parmi les images de la séquence, vers un second emplacement géographique où ces informations peuvent être collectées et/ou traitées, notamment en vue de l'établissement d'un diagnostic ou de la constitution d'une banque de données.

Les images de la séquence d'images peuvent être affichées de manière à créer une séquence animée, ce qui présente notamment l'avantage de procurer un certain effet ludique.

Les images de la séquence d'images peuvent encore être affichées en fonction d'une action de l'observateur sur au moins un curseur d'une barre de défilement, par exemple.

Dans une réalisation particulière, on affiche une image à la fois et l'on permet à un observateur de modifier deux caractéristiques de la typologie corporelle exprimées sur cette image par action sur deux curseurs de deux barres de défilement, les deux caractéristiques en question étant par exemple le nombre de rides et la profondeur de ces dernières.

Selon un autre aspect de l'invention, la séquence d'images est générée de manière à permettre une évaluation automatique par un système informatique, celui-ci comprenant par exemple un ou plusieurs réseaux de neurones, et l'on détermine par un processus de comparaison entre des images de la séquence d'images et une image de la personne que l'on cherche à évaluer, le degré effectif de la caractéristique de la typologie corporelle correspondant à cette personne.

Dans ce cas, les images de la séquence d'images n'ont pas nécessairement à être affichée sur un écran et seuls des contenus de zones mémoire peuvent être comparés, par exemple.

De préférence, la séquence d'images est générée par calcul, par exemple au moyen d'un logiciel de morphing, à partir d'au moins une image de départ et d'une image d'arrivée correspondant respectivement à des degrés différents d'une caractéristique de la typologie corporelle, de préférence des degrés extrêmes.

Lorsqu'un logiciel de morphing est utilisé, une image de la séquence autre que l'image de départ dépend normalement de l'image précédente de la séquence, cette dépendance étant liée au calcul des images, par exemple.

D'autres logiciels peuvent être utilisés pour générer la séquence d'images, notamment un logiciel de retouche d'images.

La séquence d'images peut encore être générée à partir d'un film, dont les images concernent par exemple un même sujet maquillé différemment d'une prise de vue à l'autre ou ayant reçu un produit ayant une action progressive dans le temps sur la caractéristique de la typologie corporelle concernée, par exemple un produit tenseur dans le cas des rides.

Selon un aspect de l'invention, la séquence d'images est générée en fonction d'informations concernant la personne que l'on cherche à évaluer, par exemple le nombre de rides, son type de peau, son âge, son sexe, sa couleur des yeux, ....

Ainsi, les images de départ et d'arrivée à partir desquelles la séquence d'images est générée peuvent être choisies dans au moins une banque d'images en fonction d'informations relatives à la personne à évaluer.

La séquence d'images peut également comporter plusieurs sous-séquences mises bout à bout, chaque sous-séquence étant générée à partir d'une image de départ et d'une image d'arrivée, l'image de départ de la sous-séquence d'ordre n pouvant par exemple correspondre à l'image d'arrivée de la sous-séquence d'ordre n - 1.

Chaque image de la séquence peut être générée en temps réel ou toutes les images de la séquence peuvent être générées au préalable et mémorisées avant l'étape de sélection d'une image ou d'une suite d'images de la séquence.

Le nombre d'images dans une séquence est par exemple supérieur ou égal à 10, de préférence supérieur ou égal à 20, de préférence encore supérieur ou égal à 50. Il sera choisi en fonction de la précision souhaitée, notamment.

L'invention a encore pour objet un procédé de traitement cosmétique ou dermatologique en vue de modifier au moins une caractéristique de la typologie corporelle d'un individu, comportant les étapes suivantes :
a) générer un ensemble d'images, notamment une séquence d'images, exprimant la caractéristique de la typologie corporelle avec un degré variant progressivement,
b) identifier à l'intérieur de l'ensemble d'images, notamment de la séquence d'images, une image ou suite d'images correspondant au degré effectif de la caractéristique de la typologie corporelle de l'individu,
c) prescrire en fonction de l'image ou de la suite d'images identifiée un traitement cosmétique ou dermatologique visant à modifier le degré effectif de la caractéristique de la typologie corporelle.

A l'étape b), on peut également identifier à l'intérieur de la séquence une image ou suite d'images correspondant au degré de la caractéristique de la typologie corporelle qui est souhaité après traitement cosmétique ou dermatologique, le traitement prescrit à l'étape c) étant choisi de manière à faire évoluer la caractéristique de la typologie corporelle du degré effectif vers le degré souhaité.

Le résultat de l'évaluation peut être recueilli à distance, notamment grâce à Internet.

La prescription peut être accompagnée par l'envoi d'un formulaire de commande du produit prescrit, de préférence au moyen d'Internet également.

La prescription peut également être accompagnée par l'envoi du produit de traitement prescrit.

Le produit prescrit peut être un produit cosmétique. Par produit cosmétique, on entend un produit tel que défini par la Directive 93/35/CEE du Conseil du 14 juin 1993, modifiant la Directive 76/768/CEE. Un traitement cosmétique est un traitement non thérapeutique au moyen d'un produit cosmétique tel que défini ci-dessus.

On peut recueillir, après une période donnée de traitement au moyen du produit prescrit, le résultat d'une nouvelle évaluation de la caractéristique de la typologie corporelle concernée.

Dans ce cas, en fonction du résultat de cette nouvelle évaluation, on peut maintenir ou modifier la prescription.

Compte tenu du fait que l'évaluation peut se faire de manière précise, il est possible de mettre rapidement en évidence une amélioration même relativement minime, ce qui encourage la personne traitée à poursuivre le traitement.

Il est également possible de mettre en évidence une absence d'amélioration et donc d'amener la personne traitée à changer de traitement.

On peut maintenir ou modifier la prescription en fonction du résultat de la nouvelle évaluation.

L'invention a encore pour objet un procédé visant à évaluer sur au moins un individu l'efficacité d'un traitement cosmétique ou dermatologique, comportant les étapes suivantes :
a) générer un ensemble d'images, notamment une séquence d'images, exprimant une caractéristique de la typologie corporelle sur laquelle le traitement est supposé avoir une action, les différentes images de la séquence correspondant à différentes gradations de cette caractéristique de la typologie corporelle,
b) identifier à l'intérieur de l'ensemble d'images, notamment de la séquence d'images, ainsi générée une image ou suite d'images correspondant au degré de ladite caractéristique chez l'individu, de manière à évaluer ladite caractéristique avant traitement,
c) effectuer le traitement,
d) renouveler les étapes a) et b) de manière à évaluer ladite caractéristique après traitement,
e) comparer les évaluations avant et après traitement.

Les résultats de chaque évaluation peuvent être recueillis à distance, notamment par Internet.

L'invention a encore pour objet un procédé pour générer un panel d'utilisateurs potentiels d'un produit cosmétique ou dermatologique, comportant les étapes suivantes :
a) générer une séquence d'images représentant une caractéristique de la typologie corporelle avec des degrés variables, notamment variant de manière progressive,
b) pour chaque individu d'un groupe, permettre d'identifier à l'intérieur de la séquence, une image ou suite d'images représentant le degré effectif de la typologie corporelle chez cet individu ou le degré souhaité par cet individu,
c) sélectionner parmi les individus du groupe ceux dont l'image ou la suite d'images identifiée répond à au moins un critère prédéterminé.

Les résultats obtenus à l'étape b) peuvent être recueillis à distance, une même séquence d'images pouvant être soumise facilement à tous les individus du groupe, notamment par Internet.

Une fois le panel généré, il est possible par exemple d'envoyer aux personnes composant le panel une offre d'achat de produit, voire un échantillon de produit.

L'invention a encore pour objet un procédé d'aide à la formulation cosmétique ou dermatologique, comportant les étapes suivantes :
a) générer une séquence d'images représentant une caractéristique de la typologie corporelle sur laquelle un produit cosmétique ou dermatologique est censé avoir une action, les différentes images de la séquence correspondant à différentes gradations de cette caractéristique de la typologie corporelle,
b) identifier à l'intérieur de la séquence une image ou suite d'images correspondant au degré effectif de la caractéristique de la typologie corporelle d'au moins d'un individu, et
c) formuler le produit cosmétique ou dermatologique en fonction de l'image ou de la suite d'images identifiée pour et/ou par l'individu.

Les résultats de l'étape b) peuvent être recueillis à distance, la séquence d'images pouvant être soumise à l'individu par Internet notamment.

L'étape c) ci-dessus peut se décomposer en sous étapes suivantes :
(i) générer un panel en sélectionnant parmi les individus ayant répondu, ceux dont l'image ou la suite d'images identifiée répond à au moins un critère prédéterminé,
(ii) fabriquer le produit cosmétique ou dermatologique,
(iii) appliquer ou administrer le produit aux individus du panel,
(iv) renouveler les étapes a) et b) après une ou plusieurs applications ou administrations du produit en vue d'une nouvelle évaluation,
(v) comparer les résultats de la nouvelle évaluation avec ceux des évaluations précédentes, en vue de déterminer l'efficacité du produit,
(vi) si l'efficacité du produit est jugée insuffisante, modifier la formulation du produit et/ou sa posologie, et recommencer les étapes (iii), (iv) et (v) jusqu'à l'obtention d'une efficacité jugée suffisante.

L'invention a encore pour objet un procédé pour établir un diagnostic, en vue notamment d'un traitement cosmétique ou dermatologique, caractérisé par le fait qu'il comporte les étapes suivantes :
a) générer une séquence d'images exprimant une caractéristique de la typologie corporelle avec des degrés variables, notamment variant progressivement,
b) identifier à l'intérieur de la séquence une image ou suite d'images correspondant au degré effectif de ladite caractéristique chez un individu à diagnostiquer,
c) établir un diagnostic en fonction de l'image ou de la suite d'images identifiée.

Le résultat de l'étape b) peut être recueilli à distance, notamment par Internet.

Selon un aspect de l'invention, on affiche le résultat du diagnostic sur l'écran d'un ordinateur relié à un serveur informatique, par exemple le même écran que celui sur lequel les images de la séquence ont été visualisées, lorsque lesdites images sont affichées.

L'invention a encore pour objet un procédé pour fabriquer un atlas représentant différentes gradations d'au moins une caractéristique de la typologie corporelle, caractérisé par le fait qu'il comporte les étapes suivantes :
a) sélectionner au moins une image de départ et de préférence également au moins une image d'arrivée correspondant respectivement à des degrés différents d'une caractéristique de la typologie corporelle,
b) générer, notamment au moyen d'un logiciel de morphing, une séquence d'images intermédiaires entre ces images de départ et d'arrivée,
c) sélectionner parmi les images de la séquence au moins une image,
d) réaliser un atlas comportant au moins une image ainsi sélectionnée.

L'invention permet de réaliser des atlas très précis, puisqu'il est possible grâce à l'invention de générer très facilement autant de gradations intermédiaires que l'on veut entre deux degrés d'une caractéristique de la typologie corporelle.

L'invention a encore pour objet un atlas ainsi réalisé.

L'invention a encore pour objet un procédé pour générer une banque de données multivectorielle regroupant un ensemble de vecteurs correspondant chacun à un individu, chaque vecteur comportant au moins une composante constituée par une information représentative d'une image ou d'une suite d'images d'une séquence d'images, chaque vecteur comportant de préférence au moins deux composantes constituées par des informations représentatives d'images ou de suites d'images sélectionnées parmi deux séquences exprimant des caractéristiques de la typologie corporelle différentes.

L'invention a encore pour objet un dispositif permettant de déterminer un degré d'une caractéristique de la typologie corporelle, effectif ou souhaité, caractérisé par le fait qu'il comporte une unité programmée pour générer une séquence d'images exprimant différentes gradations d'une caractéristique de la typologie corporelle.

De préférence, ladite caractéristique varie de manière continue ou sensiblement continue au sein de ladite séquence.

Dans une réalisation particulière, un tel dispositif comporte un moyen de commande tel qu'un bouton de souris, un bouton d'action affiché sur un écran tactile ou un clavier, une commande à l'oeil ou à la voix, permettant de sélectionner une image de la séquence.

Toujours dans une réalisation particulière, le dispositif comporte des moyens de saisie de données tels qu'un clavier ou un bouton d'action affiché sur un écran tactile, pour sélectionner dans au moins une banque d'images des images de départ et d'arrivée à partir desquelles la séquence peut être générée par calcul, notamment au moyen d'un logiciel de morphing, la sélection étant effectuée en fonction d'informations communiquées par un utilisateur grâce aux moyens de saisies de données précités.

Le dispositif peut comporter un générateur d'au moins une barre de défilement et d'un curseur, permettant de commander le défilement sur un écran des images de la séquence, une seule image de la séquence à la fois étant affichée, de préférence.

Le dispositif peut également comporter un générateur de deux barres de défilement et de deux curseurs associés, chaque curseur permettant de commander la modification à l'écran d'une caractéristique de la typologie corporelle exprimée par l'image affichée, par exemple le nombre de rides pour l'un des curseurs et la profondeur des rides pour l'autre curseur.

De préférence, le dispositif comporte des moyens permettant de valider la sélection d'un degré de caractéristique de la typologie corporelle, une telle validation pouvant être effectuée par exemple au moyen d'un curseur, d'un bouton d'action, d'un bouton de souris ou d'une touche de clavier, le bouton d'action pouvant présenter diverses formes, être superposé à un curseur ou à un tableau affiché à l'écran, l'écran pouvant être tactile.

Le dispositif comporte avantageusement un indicateur apte à fournir une indication, de préférence chiffrée, représentative du degré de la caractéristique de la typologie corporelle exprimée par l'image affichée à l'écran, cette indication chiffrée étant par exemple une note proportionnelle au nombre d'images de la séquence compris entre l'image affichée et une image de la séquence correspondant à un degré prédéterminé de la caractéristique de la typologie corporelle, par exemple un degré extrême.

Le dispositif comporte avantageusement un modem permettant de communiquer avec un serveur ou un ordinateur distant.

Dans une réalisation particulière, le dispositif comporte des moyens permettant d'envoyer à un serveur des informations représentatives d'une image sélectionnée.

Le dispositif peut encore comporter des moyens tels qu'une caméra ou un scanner pour acquérir une image d'une personne ou d'un dispositif de test à évaluer et un système informatique tel qu'un réseau de neurones ou un autre dispositif d'intelligence artificielle pour comparer cette image avec les images de la séquence.

L'invention a encore pour objet, selon un autre de ses aspects, un procédé permettant le traitement, notamment cosmétique, d'une partie extérieure du corps d'un individu, ce procédé comportant les étapes suivantes :
- générer une séquence d'images simulant à des degrés divers au moins une caractéristique typologique d'une partie extérieure du corps,
- permettre d'identifier au moins une image au sein de la séquence qui correspond sensiblement à un degré de ladite caractéristique, et
- déterminer un traitement de la partie extérieure en fonction de ladite au moins une image identifiée.

L'invention a encore pour objet, selon un autre de ses aspects, un procédé permettant d'évaluer un traitement d'une partie extérieure du corps d'un individu, ce procédé comportant les étapes suivantes :
- générer une séquence d'images simulant à des degrés divers au moins une caractéristique d'une partie extérieure du corps, et
- permettre l'identification d'au moins une image de la séquence qui correspond sensiblement à un degré de ladite au moins une caractéristique chez un individu, de manière à permettre l'évaluation de ladite au moins une caractéristique avant traitement,
- causer un traitement de ladite partie extérieure du corps de l'individu,
- permettre à nouveau l'identification et l'évaluation après le traitement,
- permettre une comparaison des évaluations avant et après traitement.

L'invention a encore pour objet un appareil permettant l'évaluation d'une caractéristique de la typologie corporelle, l'appareil comportant :
- un processeur configuré pour générer une séquence d'images telle que définie plus haut, de manière à permettre la mise en oeuvre de l'un des procédés précités.

L'invention a encore pour objet un appareil permettant d'évaluer une caractéristique de la typologie d'une partie extérieure du corps d'un individu, cet appareil comportant :
- un support pouvant être lu par un ordinateur pour stocker des informations permettant de générer une séquence d'images telle que définie plus haut.

D'autres caractéristiques et avantages de la présente invention ressortiront à la lecture de la description détaillée qui va suivre, d'exemples de mise en oeuvre non limitatifs, et à l'examen du dessin annexé, sur lequel :
- les figures 1 et 2 représentent de manière très schématique un ordinateur personnel,
- la figure 3 est un schéma illustrant de manière schématique certains aspects d'un programme servant à mettre en oeuvre l'invention,
- les figures 4 à 6 sont des représentations schématiques de séquences d'images concernant différentes caractéristiques de la typologie corporelle,
- la figure 7 est un schéma en blocs illustrant un exemple de procédé conforme à l'invention,
- la figure 8 représente de manière schématique un dispositif autonome pour le conseil personnalisé de produits cosmétiques ou de soins,
- la figure 9 est une vue schématique représentant un serveur relié à une pluralité d'ordinateurs personnels, notamment par le réseau Internet,
- la figure 10 illustre la constitution d'une banque de données regroupant des informations concernant différentes caractéristiques de la typologie corporelle,
- la figure 11 illustre la détermination automatique d'une caractéristique de la typologie corporelle, et
- la figure 12 représente une image affichée à l'écran avec deux curseurs associés à deux barres de défilement respectives, chaque curseur permettant de faire varier un degré d'une caractéristique de la typologie corporelle

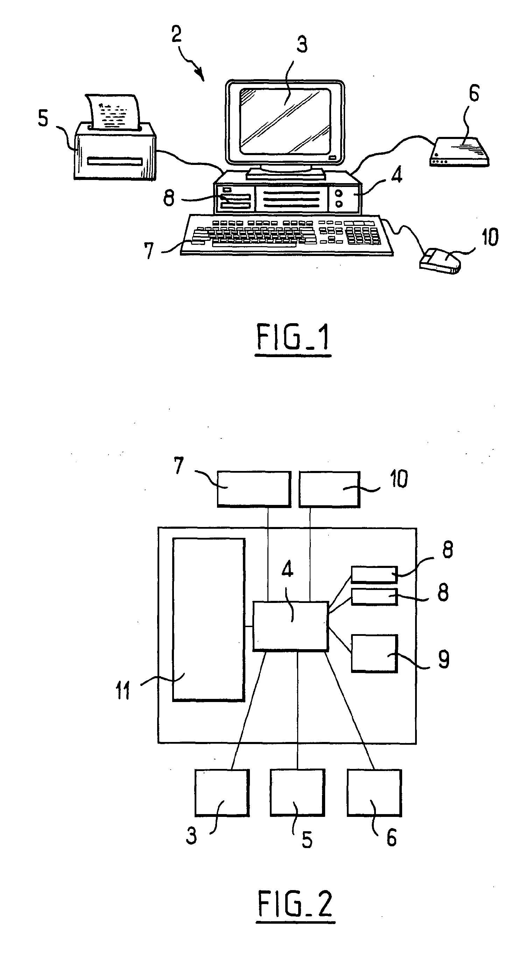

On a représenté sur les figures 1 et 2 un ordinateur personnel 2 comportant une unité centrale 4 reliée à un certain nombre de périphériques externes tels qu'un écran 3, une imprimante 5, un modem 6, un clavier 7, une souris 10, et à des périphériques internes tels que des lecteurs de cédéroms et de disquettes 8 et un disque dur 9.

L'unité centrale 4 est également reliée à une mémoire vive 11.

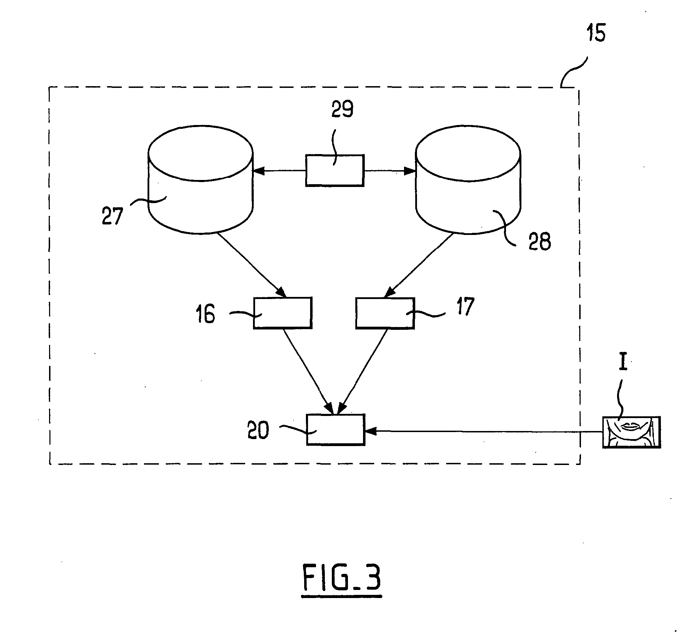

Dans un premier exemple de mise en oeuvre de l'invention, un programme 15, représenté de manière schématique à la figure 3, est chargé dans l'ordinateur personnel 2.

Ce programme 15 inclut un logiciel de morphing 20 adapté à générer une séquence d'images (ou métamorphoses) intermédiaires entre une image de départ et une image d'arrivée, contenues dans des fichiers respectifs 16 et 17.

Le programme 15 provoque, dans l'exemple décrit, l'affichage sur l'écran 3 d'une seule image de la séquence à la fois.

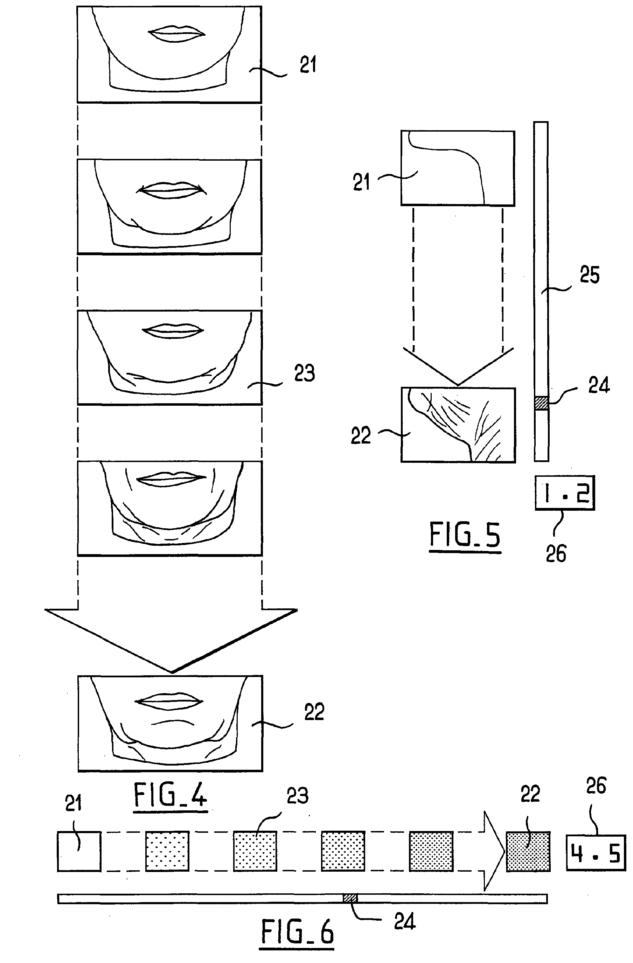

On a représenté de manière schématique à la figure 4 un exemple de séquence, dans laquelle la fermeté du bas du visage et du cou varie progressivement d'une image de départ 21 à une image d'arrivée 22, en passant par une série d'images intermédiaires 23, seules quelques images intermédiaires 23 étant apparentes sur la figure 4.

L'invention n'est pas limitée à une caractéristique particulière de la typologie corporelle et, à titre d'exemple, on a représenté sur la figure 5 les images de départ 21 et d'arrivée 22 d'une séquence d'images concernant le relâchement de la peau du cou.

Les différentes images de la séquence peuvent encore ne pas correspondre à la représentation d'une partie du corps humain mais à la représentation d'un dispositif de test, par exemple un ruban adhésif comme illustré à la figure 6.

On a représenté sur cette figure différentes quantités de sébum qui se déposent sur le ruban.

L'image de départ 21 représente le ruban vierge de tout dépôt de sébum, après application sur la peau puis retrait de cette dernière.

L'image d'arrivée 22 représente le ruban recouvert sur une partie importante de sa surface par un dépôt de sébum ou d'autres impuretés.

D'autres séquences d'images peuvent encore être générées, sans que l'on sorte du cadre de la présente invention, concernant d'autres caractéristiques de la typologie corporelle.

Le programme 15 peut être agencé pour faire défiler image par image une séquence d'images sur l'écran 3 tout en permettant à un observateur de provoquer un arrêt sur image en appuyant sur une touche du clavier 7 ou en cliquant sur un bouton de la souris 10, d'autres moyens de commande étant possibles sans que l'on sorte du cadre de la présente invention, notamment le recours à un écran tactile.

Le défilement des images 23 peut également être provoqué en fonction d'une action exercée par un observateur sur un curseur 24 d'une barre de défilement 25, comme illustré aux figures 5 et 6.

Les images de départ 21 et d'arrivée 22 peuvent être sélectionnées dans des banques d'images de départ et d'arrivée respectives 27 et 28, en fonction d'informations fournies par un moteur de sélection 29, ce dernier permettant par exemple d'afficher un ou plusieurs questionnaires et de traiter les réponses correspondantes.

Avant que les différentes images 23 d'une séquence soient générées, l'utilisateur peut ainsi renseigner le programme 15 sur certaines caractéristiques de sa typologie corporelle.

Dans le cas des rides de la patte d'oie par exemple, le programme 15 peut demander à l'utilisateur d'indiquer le nombre de rides présentes sur la zone concernée en répondant à un questionnaire.

Une image de départ ayant le nombre de rides renseigné par l'utilisateur est sélectionnée dans la banque d'images de départ 27.

Une image d'arrivée ayant le même nombre de rides est sélectionnée dans la banque d'images d'arrivée 28.

L'ordinateur fait ensuite apparaître une image de la patte d'oie comportant le nombre de rides renseigné par l'utilisateur.

La caractéristique de la typologie corporelle étant dans cet exemple la profondeur des rides, l'utilisateur a la possibilité, en agissant sur le curseur de la barre de défilement, de modifier graduellement l'image dans le sens d'une diminution de la profondeur des rides ou d'une augmentation de leur profondeur, jusqu'à ce que l'image affichée soit le reflet exact de ses propres rides.

La position du curseur sur la barre de défilement renseigne sur la profondeur des rides, cette position pouvant être repérée par exemple par une information alphanumérique 26 apparaissant sur l'écran, cette information pouvant être transmise le cas échéant à un serveur distant destiné à établir un diagnostic et/ou éventuellement à préconiser un produit ayant une action sur les rides.

L'information alphanumérique 26 peut notamment être une note proportionnelle au nombre d'images 23 de la séquence compris entre l'image affichée à l'écran et l'image de départ ou d'arrivée.

Un individu ayant une peau jeune pourrait ainsi se voir attribuer une note maximale, par exemple 10/10, et un vieillard une note minimale, par exemple 0/10.

Les différentes images 23 d'une séquence sont générées dans les exemples décrits au moyen du logiciel de morphing 20, lequel peut être de tout type connu en lui-même, réalisant par exemple par calcul des interpolations pixel à pixel de deux images, permettant ainsi de construire une série continue d'images 23 intermédiaires entre les images de départ 21 et d'arrivée 22.

A titre d'exemple de logiciels de morphing, on peut citer notamment les programmes connus sous les dénominations WINMORPH V2.1, CINEMORPH 1.2, MPMORPH 4x, TSMORPH 32, FASTMORPHER 1.03, VISIONAIRE 1.0, MORPH PLUS 1.04, V-MORPH 2.0, AGA MORPH 2.2, IMAGE MASTER 1.50R, ELASTIC DREAMS 1.01 et FANTASTIC DREAMS.

Le programme 15 peut encore comporter des moyens permettant de charger un fichier correspondant à une image I de la personne ou du dispositif de test à évaluer, obtenue au moyen d'un scanner ou d'un appareil photo numérique, par exemple.

Le programme 15 peut être agencé pour provoquer l'affichage sur l'écran 3 à la fois d'une image 23 de la séquence et de l'image I, afin de faciliter la comparaison.

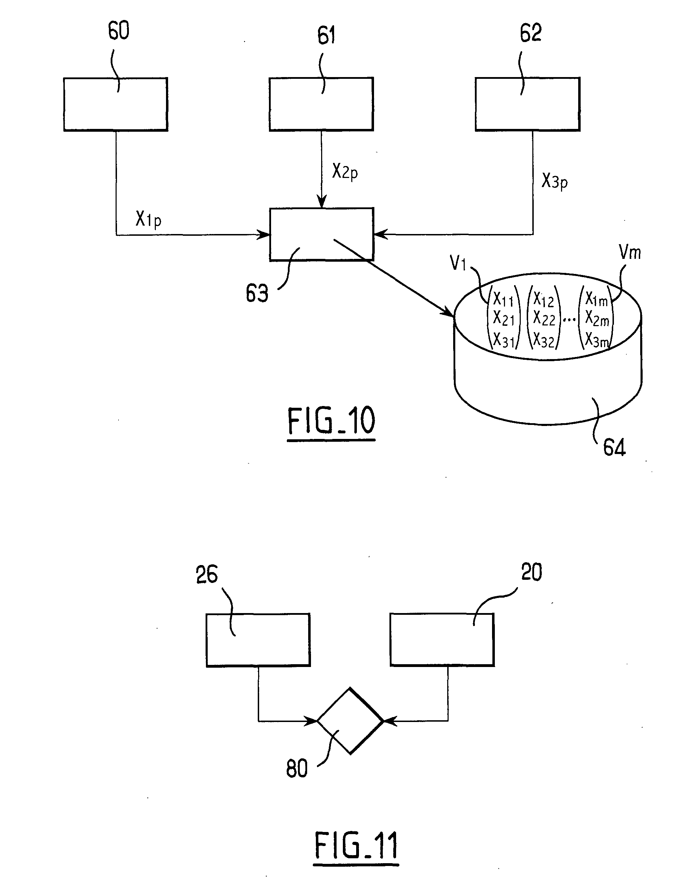

L'image I peut également être utilisée dans un moteur de comparaison 80 de type intelligence artificielle, représenté schématiquement à la figure 11, permettant de déterminer de manière automatique le degré de la caractéristique de la typologie corporelle qui correspond à la personne à évaluer.

L'ordinateur personnel 2 peut fonctionner de manière autonome, le programme 15 étant par exemple chargé à partir d'un cédérom ou d'une disquette.

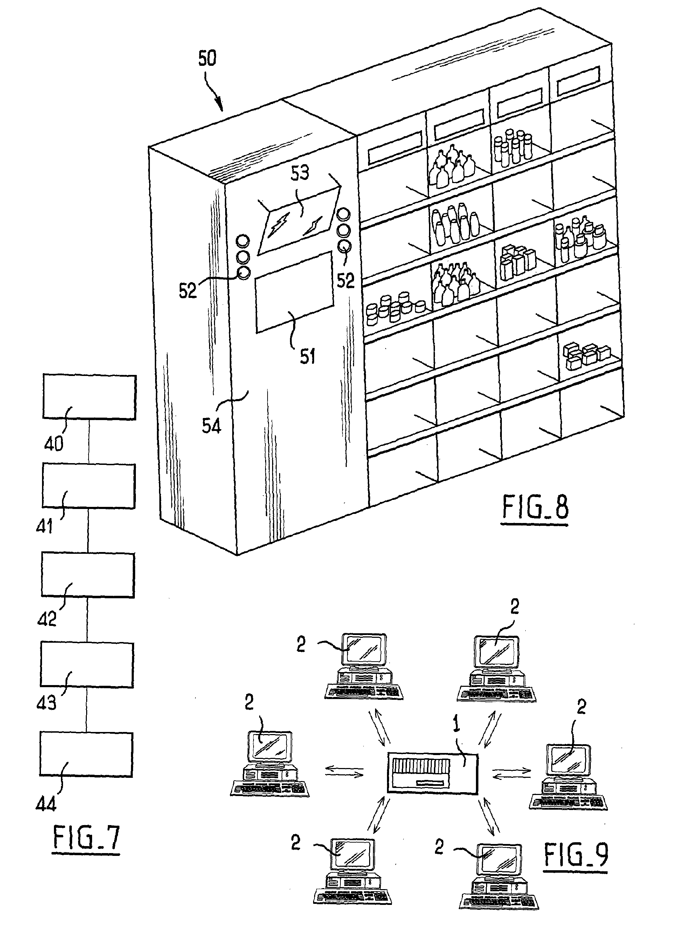

Dans ce cas, l'ordinateur 2 permet à l'utilisateur de s'auto-évaluer suivant la succession d'étapes illustrée à la figure 7.

La première étape 40 consiste par exemple pour l'utilisateur de l'ordinateur personnel 2 à répondre à différents questionnaires permettant au programme 15 de sélectionner grâce au moteur de sélection 29 l'image de départ 21 et l'image d'arrivée 22 dans les banques d'images de départ et d'arrivée respectives 28 et 29, en fonction par exemple notamment de l'âge et du sexe de l'utilisateur.

Ainsi, si la personne qui cherche à s'auto-évaluer est une jeune femme, les images de départ 21 et d'arrivée 22 seront différentes de celles qui seront sélectionnées si l'utilisateur est un homme âgé.

L'étape 41 suivante correspond à la génération d'une séquence d'images par le logiciel de morphing 20.

L'étape ultérieure 42 correspond à la sélection d'une image de la séquence, soit par arrêt sur image en appuyant sur une touche du clavier par exemple soit grâce au positionnement particulier du curseur sur la barre de défilement.

L'étape suivante 43 peut être par exemple une étape d'établissement d'un diagnostic par le programme 15, en fonction des réponses aux questionnaires et du degré de la caractéristique de la typologie corporelle précédemment évaluée.

La dernière étape 44 peut être par exemple une étape de préconisation d'un produit en fonction du diagnostic effectué à l'étape précédente 43.

Un tel produit peut être prescrit en allant rechercher dans une base de données de produits cosmétiques et de soins ceux ayant une action sur la caractéristique de la typologie corporelle qui a fait l'objet de l'évaluation et qui sont répertoriés comme étant adaptés au profil de la personne précédemment évaluée.

Le procédé de la figure 7 peut être mis en oeuvre au moyen d'un ordinateur personnel 2, comme cela vient d'être décrit, cet ordinateur étant par exemple situé au domicile de la personne à évaluer.

On ne sort pas du cadre de la présente invention lorsque l'évaluation est effectuée sur un lieu de vente de produits cosmétiques ou de soins, par exemple, comme cela va maintenant être décrit en référence à la figure 8.

Sur cette figure, on a représenté un dispositif de conseil 50 qui comporte un écran tactile 51 relié à une unité centrale non apparente, des dispositifs d'éclairage 52, un miroir 53, l'ensemble étant supporté par un bâti 54.

Le bâti 54 peut être par exemple accolé, comme illustré, à des rayonnages contenant les différents produits cosmétiques ou de soins susceptibles d'être préconisés par le dispositif de conseil 50 et répertoriés par celui-ci.

Les étapes 40 à 44 de la figure 7 peuvent être effectuées avec le dispositif de conseil 50.

Selon un aspect de l'invention, l'ordinateur personnel 2 ou le dispositif de conseil 50 est avantageusement relié, par exemple par l'intermédiaire du réseau Internet, à un serveur 1, comme illustré sur la figure 9.

Ce serveur 1 permet par exemple de télécharger sur l'ordinateur individuel 2 le programme 15 décrit précédemment.

Ainsi, l'utilisateur peut télécharger le programme 15 depuis un site Internet par exemple.

Le programme 15 permettant de générer les images 23 peut également ne pas être téléchargé mais utilisé à distance.

Dans ce cas, l'ordinateur personnel 2 relié au serveur 1 peut se comporter comme un simple terminal.

Lorsque le programme d'évaluation est consultable en ligne, l'internaute, en se connectant au site donnant accès à ce programme, peut s'évaluer ou se faire évaluer en présence d'une personne formée à cet effet et obtenir un diagnostic et, le cas échéant se voir préconiser un produit cosmétique ou de soins.

En outre, un fabricant de produits cosmétiques ou de soins peut disposer, grâce aux données susceptibles d'être collectées sur un tel site, d'informations permettant d'adapter au mieux les produits fabriqués à la typologie corporelle de sa clientèle.

En particulier, l'invention peut notamment permettre de constituer une banque de données multivectorielle regroupant des données relatives à plusieurs caractéristiques différentes de la typologie corporelle chez une même personne.

A titre d'exemple, on a représenté sur la figure 10 une image composite 63 résultant de la combinaison de trois images 60, 61 et 62 sélectionnées par une personne s'étant auto-évaluée ou ayant été évaluée, et concernant respectivement par exemple des caractéristiques de la typologie corporelle telles que la typologie du bas du visage, la typologie du milieu du visage et la typologie du haut du visage.

En collectant un ensemble d'images composites 63 ou de données représentatives de ces images, il est possible de constituer une banque de données dans laquelle chaque image composite 63 constitue en quelque sorte un portrait robot de la personne correspondante.

L'ensemble des images composites 63 peut être établi dans des conditions identiques, indépendantes de conditions de prise de vue notamment, ce qui accroît la fiabilité des données et facilite les comparaisons.

On peut ainsi constituer une banque de données multivectorielle 64 comportant une pluralité de vecteurs V₁... Vₙ ayant chacun pour composante X_{1q}, X_{2q}, X_{3q}, où q est un entier compris entre 1 et n, n étant le nombre d'individus ayant servi à constituer la banque de données.

Chaque composante est représentative d'une image identifiée dans une séquence d'images.

A titre d'exemple, pour un individu de numéro p dans le groupe, à l'image 60 identifiée peut être associée une valeur numérique X₁ₚ, à l'image 61 une valeur numérique X₂ₚ et à l'image 62 une valeur numérique X₃ₚ.

Chaque vecteur Vₚ permet de quantifier la typologie corporelle de l'individu de numéro p dans le groupe, de manière précise.

On comprend que l'invention permet une évaluation très précise d'une caractéristique de la typologie corporelle, ce qui est avantageux pour mettre en évidence l'effet d'un produit cosmétique ou de soins.

Il suffit en effet de procéder à une évaluation avant l'application ou l'administration du produit puis à une nouvelle évaluation après le traitement et comparer le résultat de ces évaluations.

Compte tenu de la précision permise par l'invention, une amélioration est rapidement mise en évidence, ce qui permet le cas échéant d'encourager la personne traitée à prolonger le traitement.

De manière analogue, une personne peut être en mesure de constater rapidement, grâce à l'invention, l'absence d'efficacité d'un traitement et être amenée à en changer.

L'invention peut permettre également de suivre l'évolution d'une pathologie afin de déterminer si un traitement devient nécessaire, par exemple.

L'invention n'est pas limitée aux caractéristiques de la typologie corporelle mentionnées plus haut et peut s'appliquer à un grand nombre de caractéristiques de la typologie corporelle, notamment la ptose du visage, la sécheresse cutanée ou l'évaluation de l'état cutané du cou, cette liste n'étant pas limitative.

Le serveur 1 décrit plus haut peut également constituer un système expert de type intelligence artificielle, agencé de manière à rendre un diagnostic de manière complètement automatique à un internaute ayant fait son auto-évaluation et ayant transmis les résultats de celle-ci au serveur 1.

L'invention n'est pas limitée à la détermination d'une seule caractéristique de la typologie corporelle à la fois.

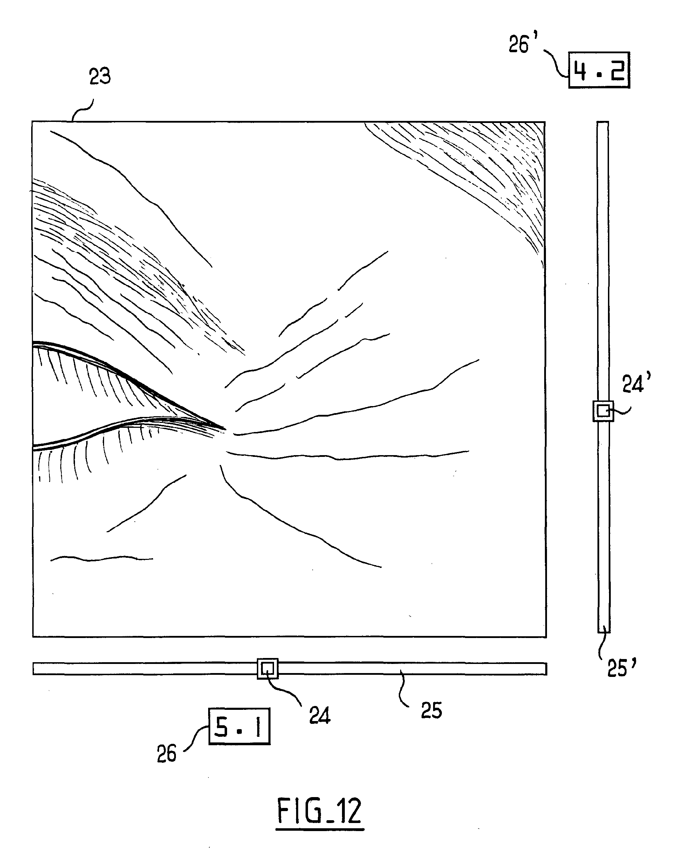

On a représenté à titre d'exemple à la figure 12, une image 23 associée à deux curseurs 24 et 24' de deux barres de défilement 25 et 25', ces curseurs 24 et 24' permettant de modifier respectivement la profondeur des rides et le nombre de rides.

Des informations numériques 26 et 26' représentatives chacune du degré de la caractéristique de la typologie corporelle correspondante sont associées aux curseurs.

En déplaçant les curseurs 24 et 24', l'utilisateur peut changer le nombre de rides et/ou la profondeur de ceux-ci.

Les différents exemples qui viennent d'être donnés peuvent s'appliquer à l'auto-évaluation.

L'évaluation peut encore être effectuée par une conseillère beauté, sur un stand de vente de produits cosmétiques ou de soins ou dans un salon d'esthétique par exemple, cette conseillère beauté comparant les différentes images apparaissant sur l'écran avec la personne à évaluer.

On peut utiliser d'autres logiciels de morphing pour générer les images d'une séquence, voire des moyens cinématographiques et/ou de traitement de signaux permettant par exemple d'agir sur le contraste ou le niveau de gris ou de certaines couleurs d'une image.

On peut choisir de n'afficher, en fonction du positionnement d'un curseur par exemple, qu'une image toutes les x images de la séquence afin de rendre plus marqué et plus rapide la modification d'une caractéristique de la typologie corporelle lors du déplacement du curseur ou faciliter la détermination de deux caractéristiques de la typologie corporelle à la fois.

Plusieurs images exprimant différentes caractéristiques de la typologie corporelle peuvent être affichées simultanément à l'écran, par exemple une image du bas du visage et une image du haut du visage.

Les séquences peuvent être construites à partir de sous-séquences.

Une image de départ d'une sous-séquence d'ordre n peut être constituée par une image d'arrivée d'une sous-séquence d'ordre n - 1, les images de départ et/ou d'arrivée des sous-séquences d'ordre n et n - 1 exprimant au moins une même caractéristique de la typologie corporelle et éventuellement une autre caractéristique.

Ainsi, dans le cas de la figure 12, les images de départ et d'arrivée des sous-séquences d'ordre n et n - 1 se rapportant à la profondeur des rides peuvent servir également d'images de départ ou d'arrivée pour des sous-séquences se rapportant au nombre de rides.

## Revendications

1. Procédé permettant de déterminer le degré effectif et/ou souhaité d'une caractéristique de la typologie corporelle d'un individu, **caractérisé par le fait qu'**il comporte les étapes suivantes :
a) générer une séquence d'images (21, 22, 23) exprimant ladite caractéristique avec un degré variable, notamment variant progressivement, et
b) permettre d'identifier à l'intérieur de la séquence, au moins une image ou suite d'images correspondant au degré effectif et/ou au degré souhaité de ladite caractéristique.

2. Procédé selon la revendication 1, **caractérisé par le fait que** les images de la séquence correspondent à différents degrés de la caractéristique de la typologie corporelle selon une progression continue ou sensiblement continue.

3. Procédé selon la revendication 1, **caractérisé par le fait que** la séquence d'images (21, 22, 23) est générée sur un ordinateur personnel (2).

4. Procédé selon l'une des trois revendications précédentes, **caractérisé par le fait que** la séquence d'images (21, 22, 23) est générée au moyen d'un serveur (1).

5. Procédé selon la revendication 1, **caractérisé par le fait que** la séquence d'images (21, 22, 23) est générée sur un dispositif de conseil (50), notamment un dispositif de conseil (50) présent sur un lieu de vente.

6. Procédé selon l'une quelconque des revendications précédentes, **caractérisé par le fait que** les images (21, 22, 23) de la séquence d'images sont affichées de manière à créer une séquence animée.

7. Procédé selon l'une quelconque des revendications 1 à 5, **caractérisé par le fait que** les images (21, 22, 23) de la séquence d'images sont affichées en fonction d'une action de l'observateur, notamment sur un curseur (24 ; 24') d'une barre de défilement (25 ; 25').

8. Procédé selon l'une quelconque des revendications précédentes, **caractérisé par le fait que** les images (21, 22, 23) de la séquence d'images sont affichées sur un écran (3) et présentées à une personne afin de permettre à celle-ci de sélectionner une image (23), et **par le fait que** l'on génère une information représentative de l'image sélectionnée.

9. Procédé selon la revendication précédente, **caractérisé par le fait que** l'information représentative de l'image sélectionnée comporte un nombre (26 ; 26').

10. Procédé selon l'une des deux revendications immédiatement précédentes, **caractérisé par le fait que** l'on recueille l'information représentative de l'image (23) sélectionnée à distance.

11. Procédé selon la revendication précédente, **caractérisé par le fait qu'**il comporte les étapes consistant à afficher en un premier emplacement géographique des images (21, 22, 23) d'une séquence d'images puis à transférer sous un ou plusieurs protocoles de communication, notamment sous protocole IP, des informations représentatives d'une sélection opérée parmi les images (21, 22, 23) de la séquence, vers un second emplacement géographique où ces informations sont collectées et/ou traitées, notamment en vue de l'établissement d'un diagnostic ou de la constitution d'une banque de données.

12. Procédé selon la revendication 1, **caractérisé par le fait que** la séquence d'images est générée de manière à permettre une évaluation automatique par un système informatique (80), et **par le fait que** l'on détermine, par un processus de comparaison entre des images (23) de la séquence d'images et une image (I) de la personne que l'on cherche à évaluer, le degré effectif de la caractéristique de la typologie corporelle correspondant à cette personne.

13. Procédé selon l'une quelconque des revendications précédentes, **caractérisé par le fait que** la séquence d'images est générée par calcul, de préférence au moyen d'un logiciel de "morphing" (20), à partir d'au moins d'une image de départ (21) et d'une image d'arrivée (22), correspondant respectivement à des degrés différents d'une caractéristique de la typologie corporelle.

14. Procédé selon l'une quelconque des revendications précédentes, **caractérisé par le fait que** la séquence d'images est générée en fonction d'informations concernant la personne que l'on cherche à évaluer.

15. Procédé selon la revendication 13, **caractérisé par le fait que** les images de départ et d'arrivée sont choisies dans au moins une banque d'images (27, 28) en fonction d'informations relatives à la personne à évaluer.

16. Procédé selon l'une quelconque des revendications précédentes, **caractérisé par le fait que** la séquence d'images comporte plusieurs sous-séquences mises bout à bout, chaque sous-séquence étant générée à partir d'une image de départ et d'une image d'arrivée, l'image de départ de la sous-séquence d'ordre n correspondant de préférence à l'image d'arrivée de la sous-séquence d'ordre n - 1.

17. Procédé selon l'une quelconque des revendications précédentes, **caractérisé par le fait que** l'on affiche une image (23) et l'on permet à un observateur de modifier deux caractéristiques de la typologie corporelle exprimées par cette image par action sur deux curseurs (24 ; 24') de deux barres de défilement (25 ; 25').

18. Procédé selon l'une quelconque des revendications précédentes, **caractérisé par le fait que** le nombre d'images d'une séquence est supérieur ou égal à 10, de préférence supérieur ou égal à 20, de préférence encore supérieur ou égal à 50.

19. Procédé de traitement cosmétique en vue de modifier au moins une caractéristique de la typologie corporelle d'un individu, **caractérisé par le fait qu'**il comporte les étapes suivantes :
a) générer une séquence d'images exprimant la caractéristique de la typologie corporelle avec un degré variable, notamment avec un degré variant progressivement,
b) permettre d'identifier à l'intérieur de la séquence d'images, une image ou suite d'images correspondant au degré effectif de la caractéristique de la typologie corporelle de l'individu,
c) prescrire en fonction de l'image ou de la suite d'images identifiée un traitement cosmétique visant à modifier le degré effectif de la caractéristique de la typologie corporelle.

20. Procédé selon la revendication 19, **caractérisé par le fait que** l'on permet d'identifier à l'intérieur de la séquence une image ou suite d'images correspondant au degré de la caractéristique de la typologie corporelle qui est souhaité après traitement cosmétique, le traitement prescrit à l'étape c) étant choisi de manière à faire évoluer la caractéristique de la typologie corporelle du degré effectif vers le degré souhaité.

21. Procédé selon la revendication 19 ou 20, **caractérisé par le fait que** l'on recueille à distance le résultat de l'évaluation, notamment grâce à Internet.

22. Procédé selon l'une quelconque des revendications 19 à 21, **caractérisé par le fait que** la prescription est accompagnée par l'envoi du produit de traitement prescrit.

23. Procédé selon l'une quelconque des revendications 19 à 22, **caractérisé par le fait que** l'on recueille, après une période donnée de traitement au moyen du produit prescrit, les résultats d'une nouvelle évaluation de la caractéristique de la typologie corporelle concernée.

24. Procédé selon la revendication précédente, **caractérisé par le fait que** l'on maintient ou que l'on modifie la prescription en fonction du résultat de la nouvelle évaluation.

25. Procédé visant à évaluer sur au moins un individu l'efficacité d'un traitement cosmétique, **caractérisé par le fait qu'**il comporte les étapes suivantes :
a) générer une séquence d'images exprimant une caractéristique de la typologie corporelle sur laquelle le traitement est supposé avoir une action, les différentes images de la séquence correspondant à différentes gradations de cette caractéristique de la typologie corporelle,
b) identifier à l'intérieur de la séquence d'images ainsi générée une image ou suite d'images correspondant au degré de ladite caractéristique chez l'individu, de manière à évaluer ladite caractéristique avant traitement,
c) effectuer le traitement,
d) renouveler les étapes a) et b) de manière à évaluer ladite caractéristique après traitement,
e) comparer les évaluations avant et après traitement.

26. Procédé selon la revendication précédente, **caractérisé par le fait que** l'on recueille à distance les résultats de chaque évaluation.

27. Procédé pour générer un panel d'utilisateurs potentiels d'un produit cosmétique ou dermatologique, **caractérisé par le fait qu'**il comporte les étapes suivantes :
a) générer une séquence d'images représentant une caractéristique de la typologie corporelle avec des degrés variables, notamment des degrés variant de manière progressive,
b) pour chaque individu d'un groupe, permettre d'identifier à l'intérieur de la séquence, une image ou suite d'images représentant le degré effectif de la typologie corporelle chez cet individu ou le degré souhaité par cet individu.
c) sélectionner parmi les individus du groupe ceux dont l'image ou la suite d'images identifiée répond à au moins un critère prédéterminé.

28. Procédé d'aide à la formulation cosmétique, **caractérisé par le fait qu'**il comporte les étapes suivantes :
a) générer une séquence d'images exprimant une caractéristique de la typologie corporelle sur laquelle un produit cosmétique est censé avoir une action, les différentes images de la séquence correspondant à différentes gradations de cette caractéristique de la typologie corporelle,
b) identifier à l'intérieur de la séquence une image ou suite d'images correspondant au degré effectif de la caractéristique de la typologie corporelle d'au moins d'un individu, et
c) formuler le produit cosmétique en fonction de l'image ou de la suite d'images identifiée pour et/ou par l'individu.

29. Procédé selon la revendication 28, **caractérisé par le fait que** l'on recueille les résultats de l'étape b) à distance.

30. Procédé selon la revendication 28 ou 29, **caractérisé par le fait que** l'étape c) ci-dessus se décompose en sous étapes suivantes :
(i) générer un panel en sélectionnant parmi les individus ayant répondu, ceux dont l'image ou la suite d'images identifiée répond à au moins un critère prédéterminé,
(ii) fabriquer le produit cosmétique,
(iii) appliquer ou administrer le produit aux individus du panel,
(iv) renouveler les étapes a) et b) après une ou plusieurs applications ou administrations du produit en vue d'une nouvelle évaluation,
(v) comparer les résultats de la nouvelle évaluation avec ceux des évaluations précédentes, en vue de déterminer l'efficacité du produit,
(vi) si l'efficacité du produit est jugée insuffisante, modifier la formulation du produit et/ou sa posologie, et recommencer les étapes (iii), (iv) et (v) jusqu'à l'obtention d'une efficacité jugée suffisante.

31. Procédé pour établir un diagnostic, **caractérisé par le fait qu'**il comporte les étapes suivantes :
a) générer une séquence d'images exprimant une caractéristique de la typologie corporelle avec un degré variable, notamment un degré variant progressivement,
b) permettre d'identifier à l'intérieur de la séquence une image ou suite d'images correspondant au degré effectif de ladite caractéristique chez un individu à diagnostiquer,
c) établir un diagnostic cosmétique en fonction de l'image ou de la suite d'images identifiée.

32. Procédé selon la revendication 31, **caractérisé par le fait que** l'on recueille les résultats de l'étape b) à distance.

33. Procédé selon la revendication précédente, **caractérisé par le fait que** l'on affiche le résultat du diagnostic sur l'écran d'un ordinateur relié à un serveur informatique.

34. Procédé pour fabriquer un atlas représentant différentes gradations d'au moins une caractéristique de la typologie corporelle, **caractérisé par le fait qu'**il comporte les étapes suivantes :
a) sélectionner au moins une image de départ et de préférence également au moins une image d'arrivée correspondant respectivement à des degrés différents d'une caractéristique de la typologie corporelle,
b) générer, notamment au moyen d'un logiciel de "morphing", une séquence d'images intermédiaires entre ces images de départ et d'arrivée,
c) sélectionner parmi les images de la séquence au moins une image,
d) réaliser un atlas comportant au moins une image ainsi sélectionnée.

35. Procédé pour générer une banque de données multivectorielle regroupant un ensemble de vecteurs correspondant chacun à un individu, chaque vecteur (V₁, ..., Vₙ) comportant au moins une composante (X_{1q}, X_{2q}, X_{3q}) constituée par une information représentative d'une image ou d'une suite d'images d'une séquence d'images identifiée selon le procédé de la revendication 1, chaque vecteur comportant de préférence au moins deux composantes constituées par des informations représentatives d'images ou de suites d'images sélectionnées parmi deux séquences exprimant des caractéristiques de la typologie corporelle différentes.

36. Utilisation d'un procédé selon l'une quelconque des revendications précédentes avec l'une des caractéristiques de la typologie corporelle suivantes, à l'exclusion de toute application thérapeutique :
- caractéristiques relatives au vieillissement telles que nombre et profondeur des rides, relâchement de la peau, notamment du cou, nombre et profondeur des plis, notamment du cou, plissement de la peau, profondeur des cernes, taux de cellulite, ptose,
- caractéristiques physiologiques ou morphologiques telles que nombre et taille des cornéoparticules de la peau, taux de sécrétion de sébum, taux de sécrétion de la sueur, sécheresse de la peau ou des lèvres, longueur, courbure ou densité des cils ou des cheveux, défaut de pigmentation, densité de points noirs, de boutons d'acné, de couperose, grains de la peau, contour des lèvres,
- couleur de la peau ou des cheveux.

37. Dispositif (1 ; 2 ; 50) permettant de déterminer un degré d'une caractéristique de la typologie corporelle afin de mettre en oeuvre le procédé tel que défini dans l'une quelconque des revendications 1 à 18, **caractérisé par le fait qu'**il comporte une unité programmée pour générer une séquence d'images (21, 22, 23) exprimant différentes gradations d'une caractéristique de la typologie corporelle.

38. Dispositif selon la revendication 37, **caractérisé par le fait que** ladite caractéristique varie de manière continue ou sensiblement continue au sein de ladite séquence.

39. Dispositif selon la revendication 37 ou 38, **caractérisé par le fait qu'**il comporte un moyen de commande tel qu'un bouton de souris (10) ou un bouton d'action affiché sur un écran tactile (51) ou un clavier (7), une commande à l'oeil ou à la voix, permettant de sélectionner une image de la séquence.

40. Dispositif selon l'une des revendications 37 ou 39, **caractérisé par le fait qu'**il comporte des moyens de saisie de données tels qu'un clavier (7) ou un bouton d'action affiché sur un écran tactile (51), pour sélectionner dans au moins une banque d'images (27, 28) des images de départ (21) et d'arrivée (22) à partir desquelles la séquence est générée par calcul, notamment au moyen d'un logiciel de « morphing » (20), en fonction d'informations communiquées par un utilisateur grâce aux moyens de saisie de données.

41. Dispositif selon l'une des revendications 37 à 40, **caractérisé par le fait qu'**il comporte un générateur d'au moins une barre de défilement (25 ; 25') et d'un curseur (24 ; 24'), permettant de commander le défilement sur un écran (3 ; 50) des images de la séquence, une seule image de la séquence étant affichée à la fois.

42. Dispositif selon l'une quelconque des revendications 37 à 41, **caractérisé par le fait qu'**il comporte des moyens permettant d'envoyer à un serveur des informations représentatives d'une image sélectionnée.

43. Dispositif selon l'une quelconque des revendications 37 à 42, **caractérisé par le fait qu'**il comporte des moyens permettant d'envoyer à un serveur (1) des informations (26 ; 26') représentatives d'une image sélectionnée.

44. Dispositif selon l'une quelconque des revendications 37 à 43, **caractérisé par le fait qu'**il comporte des moyens tels qu'une caméra ou un scanner pour acquérir une image (I) d'une personne ou d'un dispositif de test à évaluer et un système informatique (80) pour comparer cette image (I) avec les images (23) de la séquence.

45. Dispositif selon l'une quelconque des revendications 37 à 44, **caractérisé par le fait qu'**il comporte un générateur de deux barres de défilement et de deux curseurs associés, chaque curseur permettant de commander la modification à l'écran d'une caractéristique de la typologie corporelle exprimée par l'image affichée.

46. Dispositif selon l'une quelconque des revendications 37 à 45, **caractérisé par le fait qu'**il comporte des moyens permettant de valider la sélection d'un degré de la caractéristique de la typologie corporelle.

47. Dispositif selon l'une quelconque des revendications 37 à 46, **caractérisé par le fait qu'**il comporte un indicateur apte à fournir une indication (26 ; 26'), de préférence chiffrée, représentative du degré de la caractéristique de la typologie corporelle exprimée par l'image affichée à l'écran.

48. Procédé de traitement cosmétique, d'une partie extérieure du corps d'un individu, ce procédé comportant les étapes suivantes :
- générer une séquence d'images simulant à des degrés divers au moins une caractéristique typologique d'une partie extérieure du corps,
- permettre d'identifier au moins une image au sein de la séquence qui correspond sensiblement à un degré de ladite caractéristique, et
- déterminer un traitement de la partie extérieure en fonction de ladite au moins une image identifiée.

49. Procédé permettant d'évaluer un traitement cosmétique d'une partie extérieure du corps d'un individu, ce procédé comportant les étapes suivantes :
- générer une séquence d'images simulant à des degrés divers au moins une caractéristique d'une partie extérieure du corps, et
- permettre l'identification d'au moins une image de la séquence qui correspond sensiblement à un degré de ladite au moins une caractéristique chez un individu, de manière à permettre l'évaluation de ladite au moins une caractéristique avant traitement,
- causer un traitement cosmétique de ladite partie extérieure du corps de l'individu,
- permettre à nouveau l'identification et l'évaluation après le traitement,
- permettre une comparaison des évaluations avant et après traitement.

50. Appareil permettant l'évaluation d'une caractéristique de la typologie corporelle, comportant :
- un processeur configuré pour générer une séquence d'images telle que définie dans la revendication 1, de manière à permettre la mise en oeuvre de l'un des procédés définis dans l'une des revendications précédentes de procédé.

51. Appareil permettant d'évaluer une caractéristique de la typologie d'une partie extérieure du corps d'un individu, cet appareil comportant :
- un support pouvant être lu par un ordinateur pour stocker des informations permettant de générer une séquence d'images telle que définie dans la revendication 1.
